# EUROPÄISCHE PATENTANMELDUNG

(11) **EP 1 432 010 A2**
(43) Veröffentlichungstag der Anmeldung: **23.06.2004**
(21) Anmeldenummer: 02028575.5
(22) Anmeldetag: 20.12.2002
(51) Int. Cl.: H01J 61/44

(54) **UV-Leuchtstoffröhre**

(30) Priorität: 20.12.2002 DE 10162826
(71) Anmelder: UV-Power Licht GmbH, 49716 Meppen (DE)
(72) Erfinder: Schaffron, Günter, 49716 Meppen (DE)
(74) Vertreter: Patentanwälte Hauck, Graalfs, Wehnert, Döring, Siemons, Schildberg

(57) **Zusammenfassung**

Es wird eine UV-Leuchtstoffröhre zur Verwendung in Solarien u.dgl., die als Niederdruckröhre ausgebildet ist, beschrieben. Die Leuchtstoffröhre enthält einen Grundleuchtstoff und einen zugemischten Leuchtstoff. Je nach Art des zugemischten Leuchtstoffes können Leuchtstoffröhren vom Typ 3 oder Typ 4 der Euronorm 60335-2-27 erhalten werden.

## Beschreibung

Die vorliegende Erfindung betrifft eine UV-Leuchtstoffröhre zur Verwendung in Solarien u.dgl., die als Niederdruckröhre ausgebildet ist.

Derartige UV-Niederdruckröhren sind bekannt. Sie geben UV-A-Strahlung und UV-B-Strahlung ab. Beide Strahlungen lösen, vom Körper aufgenommen, folgende photobiologische Prozesse und/oder photochemische Prozesse aus:
UV-B
   - Melaninaktivierung in den Melanozyten
   - Verdickung der obersten Hautschicht (Epidermis) = Lichtschwiele
   - Sonnenbrand (Erythem)
   - Vitamin D-Aktivierung
UV-A
   - Bräunung, direkte Pigmentdunklung
   - Hautalterung
   - phototoxische/photoallergische Reaktionen der Haut
   - Wärmeentwicklung

Die Intensität der UV-A-Strahlung und der UV-B-Strahlung wird als Bestrahlungsstärke gemessen. Die Bestrahlungsstärke ist das Verhältnis vom Strahlenfluß zu einer bestrahlten Fläche. Die Meßeinheit ist W/m².

Bei der biologischen Bestrahlungsstärke handelt es sich um die Strahlung, die auf der menschlichen Haut eine entsprechende Wirkung erzielt. Hierbei unterscheidet man drei verschiedene Wirkungen:
Eₚᵢ = Direkt- oder Sofortpigmentierung
Eₚₚ = verzögerte Tiefenpigmentierung
Eₑᵣ = erythemwirksame Bestrahlungsstärke

Die erythemwirksame Bestrahlungsstärke ist wohl der wichtigste Wert, da sie die Besonnungszeit bestimmt. Sie erzeugt auch den Sonnenbrand auf der Haut. Die erythemwirksame Strahlung liegt in einem Wellenbereich von 280 nm-400 nm. Der Hauptanteil dieser Strahlung liegt im UV-B-Bereich von 280 nm-315 nm.

Die verzögerte Tiefenpigmentierung steht im direkten Zusammenhang mit der erythemwirksamen Bestrahlungsstärke. Je höher der Wert der verzögerten Tiefenpigmentierung ist, desto höher ist auch der Wert der erythemwirksamen Bestrahlungsstärke und somit auch die Sonnenbrandgefahr. Die Strahlung der verzögerten Tiefenpigmentierung sorgt für die Pigmentneubildung in der Haut und für eine lang anhaltende tiefe Bräune. Diese Strahlung liegt im Wellenbereich von 285 nm-440 nm. Der Hauptanteil liegt im UV-B-Bereich von 280 nm-315 nm.

Die Direkt- oder Sofortpigmentierung dunkelt die bereits vorhandenen Hautpigmente. Die Strahlung liegt im Wellenlängenbereich von 300 nm-440 nm. Der Hauptanteil liegt im UV-A-Bereich von 315 nm-400 nm.

In bezug auf UV-Niederdruckröhren existiert die Euronorm 60335-2-27. Hiernach dürfen UV-Geräte keine Strahlung in gefährlichem Maß emittieren, und die wirksame Bestrahlungsstärke muß den in der nachfolgenden Tabelle festgelegten Werten entsprechen.

| UV-Typ | Wirksame Bestrahlungsstärke W/m² | |
|---|---|---|
| | 250 < λ ≤ 320 nm | 320 < λ ≤ 400 nm |
| 1 | <0.0005 | ≥ 0.15 |
| 2 | 0,0005 bis 0,15 | ≥ 0.15 |
| 3 | < 0,15 | < 0.15 |
| 4 | ≥ 0,15 | <0.15 |
| λ ist die Wellenlänge der Strahlung. | | |

Gemäß der obigen Tabelle gibt es in bezug auf die Grenzen der wirksamen Bestrahlungsstärke vier UV-Typen.

Der vorliegenden Erfindung liegt die Aufgabe zugrunde, eine als Niederdruckröhre ausgebildete UV-Leuchtstoffröhre zu schaffen, die nach Euronorm 60335-2-27 den Anforderungen einer Niederdruckröhre vom Typ 3 oder Typ 4 genügt, jedoch trotzdem ein weitgehend optimales Spektrum hinsichtlich der indirekten Pigmentierung (Erythemwirksamkeit) bietet.

Diese Aufgabe wird erfindungsgemäße dadurch gelöst, daß die Niederdruckröhre einen Grundleuchtstoff, dessen erythemwirksame Bestrahlungsstärke Eₑᵣ bei einer Wellenlänge λ von über 320 nm s 0,15 W/m² beträgt und einen zugemischten Leuchtstoff enthält, der die Eigenschaft besitzt, grundsätzlich nur bis zu einer wellenlänze λ von max. 320 nm eine UV-Strahlung zu erzeugen.

Die erfindungsgemäß ausgebildete UV-Leuchtstoffröhre enthält somit zwei unterschiedliche Leuchtstoffe (Phosphore), die miteinander vermischt sind. Der Grundleuchtstoff besitzt hierbei bei gleicher Energiezufuhr immer die gleichen Eigenschaften. Wenn die Leuchtstoffröhre in ihren Eigenschaften verändert werden soll, so erfolgt dies über eine Veränderung des zugemischten Leuchtstoffes.

Je nachdem, welcher Leuchtstoff unter 320 nm zugemischt wird, ergibt sich eine Lampe vom Typ 3 oder vom Typ 4 der Euronorm 60335-2-27. Wie erwähnt, wird bei gleicher Energiezufuhr die Eigenschaft der Leuchtstoffröhre nur mit dem zugemischten Leuchtstoff verändert.

Was den Grundleuchtstoff anbetrifft, so darf dessen erythemwirksame Bestrahlungsstärke über 320 nm den Wert von 0,15 W/m² grundsätzlich nicht überschreiten. Damit sind die entsprechenden Bedingungen für den UV-Typ 3 oder 4 der vorstehend genannten Euronorm erfüllt.

Der zugemischte Leuchtstoff muß die Eigenschaft besitzen, grundsätzlich nur bis max. 320 nm eine UV-Strahlung zu erzeugen. Damit sind ebenfalls die Bedinungen des UV-Typs 3 oder 4 der genannten Euronorm erfüllt. Je nach Art dieses zugemischten Leuchtstoffes läßt sich nunmehr eine Niederdruckröhre vom Typ 4 oder vom Typ 3 erzielen. Wird ein Leuchtstoff zugemischt, der keine Begrenzung der UV-Werte aufweist, so ergibt sich gemäß Euronorm ein UV-Typ 4. Der UV-Typ 3 wird erreicht, wenn der zugemischte Leuchtstoff einen erythemwirksame Bestrahlungsstärke bei einer Wellenlänge λ von unter 320 nm von max. 0,15 W/m² aufweist.

Mit dem zugemischten Leuchtstoff wird somit die Erythemwirksamkeit und der UV-B-Wert der UV-Niederdruckröhren gesteuert.

Die zugeführte Leistung beläuft sich je nach Niederdruckröhre auf einen Wert zwischen 10 und 160 Watt, in Ausnahmefällen auf einen Wert bis 180 Watt.

In Weiterbildung der Erfindung ist der E_{UVA}-Wert des Grundleuchtstoffes so hoch wie möglich. Dieser Wert E_{UVA} ergibt die energetische Bestrahlungsstärke, d.h. die Gesamtstrahlung, die auf eine bestrahlte Fläche auftrifft, für die UV-A-Strahlung wieder und wird in W/m² gemessen. Vorzugsweise ist auch der Eₚᵢ-Wert des Grundleuchtstoffes so hoch wie möglich. Der Wert Eₚᵢ ergibt die Direkt- oder Sofortpigmentierung in W/m² wieder.

Die zulässige Energiezufuhr der erfindungsgemäß ausgebildeten UV-Leuchtstoffröhre beträgt vorzugsweise 10 bis 180 W, insbesondere 10 bis 160 W.

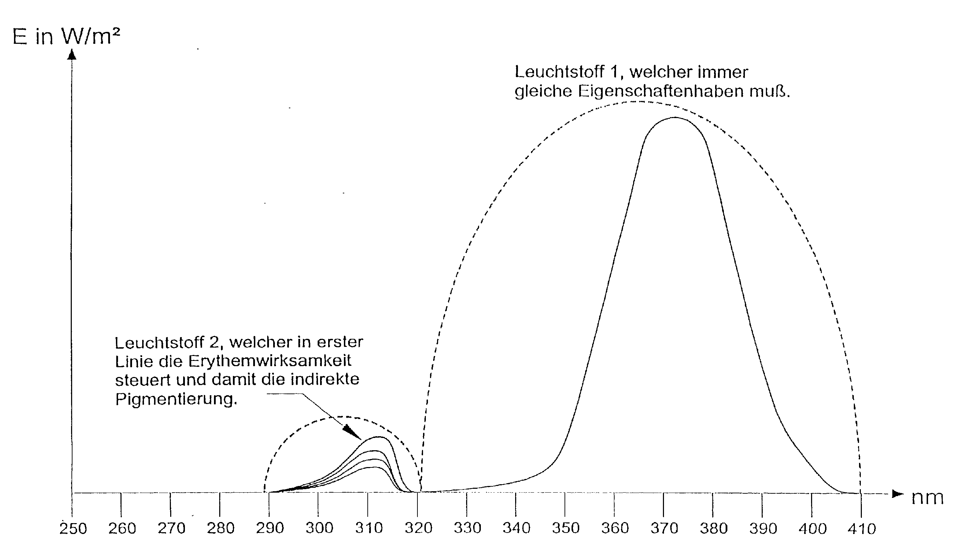

Die Erfindung wird nachfolgend anhand von Ausführungsbeispielen in Verbindung mit der Zeichnung im einzelnen erläutert. Die einzige Figur zeigt in einem Diagramm die Abhängigkeit der Bestrahlungsstärke E von der Wellenlänge bei verschiedenen Leuchtstoffröhren.

Zum Erhalt einer UV-Niederdruckröhre vom Typ 4 nach Euronorm 60335-2-27 wurde ein Grundleuchtstoff verwendet, dessen erythemwirksame Bestrahlungsstärke über 320 nm unter 0,15 W/m² lag. Diesem Leuchtstoff wurde ein zweiter Leuchtstoff zugemischt, der nur bis max. 320 nm eine UV-Strahlung erzeugte.

Zur Herstellung einer UV-Niederdruckröhre vom Typ 3 wurde der gleiche Grundleuchtstoff verwendet. Der zugemischte Leuchtstoff hatte ebenfalls die Eigenschaft, nur bis max. 320 nm eine UV-Strahlung zu erzeugen. Bie diesem Leuchtstoff lag jedoch die Erythemwirksamkeit unter 320 nm unter 0,15 W/m².

Die in beiden Fällen erhaltenen Leuchtstoffröhren wiesen gute Ergebnisse hinsichtlich der indirekten Pigmentierung und der Erythemwirksamkeit auf und entsprachen den Typen 4 und 3 der Euronorm 60335-2-27.

Die Figur zeigt ein Diagramm, in dem auf der Ordinate die Bestrahlungsstärke E in W/m² und auf der Abszisse die Wellenlänge aufgetragen ist. Der rechte Peak der erhaltenen Kurve entspricht dem Grundleuchtstoff, während die linken Peaks dem zugemischten Leuchtstoff entsprechen. Je nachdem, welcher Leuchtstoff unter 320 nm zugemischt wird, ergibt sich eine Lampe vom Typ 3 oder vom Typ 4. Bei gleicher Energiezufuhr werden die Eigenschaften der Niederdruckröhre nur mit dem zugemischten Leuchtstoff verändert.

## Patentansprüche

1. UV-Leuchtstoffröhre zur Verwendung in Solarien u.dgl., die als Niederdruckröhre ausgebildet ist, **dadurch gekennzeichnet, daß** sie einen Grundleuchtstoff, dessen erythemwirksame Bestrahlungsstärke Eₑᵣ bei einer Wellenlänge λ von über 320 nm ≤ 0,15 W/m² beträgt, und einen zugemischten Leuchtstoff enthält, der die Eigenschaft besitzt, grundsätzlich nur bis zu einer Wellenlänge λ von max. 320 nm eine UV-Strahlung zu erzeugen.

2. UV-Leuchtstoffröhre nach Anspruch 1, **dadurch gekennzeichnet, daß** der zugemischte Leuchtstoff keine Begrenzung der UV-Werte aufweist.

3. UV-Leuchtstoffröhre nach Anspruch 1, **dadurch gekennzeichnet, daß** der zugemischte Leuchtstoff eine erythemwirksame Bestrahlungsstärke bei einer Wellenlänge λ von unter 320 nm von max. 0,15 W/m² aufweist.

4. UV-Leuchtstoffröhre nach einem der vorangehenden Ansprüche, **dadurch gekennzeichnet, daß** der E_{UVA}-Wert des Grundleuchtstoffes so hoch wie möglich ist.

5. UV-Leuchtstoffröhre nach einem der vorangehenden Ansprüche, **dadurch gekennzeichnet, daß** der Eₚᵢ-Wert des Grundleuchtstoffes so hoch wie möglich ist.

6. UV-Leuchtstoffröhre nach einem der vorangehenden Ansprüche, **dadurch gekennzeichnet, daß** die zulässige Energiezufuhr 10 bis 180 W, vorzugsweise 10 bis 160 W, beträgt.
